(19) Europäisches Patentamt
European Patent Office
Office européen des brevets

(11) Veröffentlichungsnummer: **0 283 943**
**B1**

# (12) EUROPÄISCHE PATENTSCHRIFT

(45) Veröffentlichungstag der Patentschrift: **06.06.90**

(51) Int. Cl.[5]: **C07C 9/22,** A01N 27/00

(21) Anmeldenummer: **88104304.6**

(22) Anmeldetag: **18.03.88**

(54) **Verfahren und Mittel zur Bekämpfung der Kaffeeminiermotte, insbesondere der Art Perileucoptera coffeella.**

(30) Priorität: **26.03.87 DE 3709922**

(43) Veröffentlichungstag der Anmeldung:
**28.09.88 Patentblatt 88/39**

(45) Bekanntmachung des Hinweises auf die Patenterteilung:
**06.06.90 Patentblatt 90/23**

(84) Benannte Vertragsstaaten:
**AT BE CH DE ES FR GB GR IT LI NL SE**

(56) Entgegenhaltungen:
**EP-A- 0 245 772**

**Chemical Abstracts, Band 106, Nr. 23, 8. Juni 1987, Columbus, Ohio, USA W.Francke et al.: "Identification of 5,9-dimethylheptadecane as a sex pheromone of the moth Leucoptera scitella" Seite 452, Zusammenfassung-Nr. 193 131g**
**Chemical Abstracts, Band 100, Nr. 13, 26. März 1984, Columbus, Ohio, USA K.H. Lockey "Hydrocarbons of Metriopus depressus (Haag) and Renatiella scrobipennis (Haag) (Coleoptera Tenebrionidae)" Seite 383, Zusammenfassung-Nr. 100 209b**

(73) Patentinhaber: **BASF Aktiengesellschaft,**
**Carl-Bosch-Strasse 38, D-6700 Ludwigshafen(DE)**

(72) Erfinder: **Krieg, Wolfgang, Dr., Saarstrasse 17, D-6721 Weingarten(DE)**
Erfinder: **Ernst, Hansgeorg, Dr., Bussardweg 62, D-6720 Speyer(DE)**
Erfinder: **Buschmann, Ernst, Dr., Georg-Ludwig-Krebs-Strasse 10, 6700 Ludwigshafen(DE)**
Erfinder: **Francke, Wittko, Prof. Dr., Am Vorwerksbusch, D-2057 Reinbek(DE)**
Erfinder: **Engels, Wolf, Prof. Dr., Panoramastrasse 49, D-7400 Tuebingen 7(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Kaffeeminiermotte Perileucoptera coffeella (Guer. Menev., 1842) aus der Familie der Lyonetiidae ist der wichtigste tierische Schaderreger in Kaffeekulturen. Dieser Kleinschmetterling (Perileucoptera coffeella) tritt in Kaffee anbauenden Regionen in Zentral- und Südamerika sowie auf den Westindischen Inseln und Madagaskar auf.

Nahe verwandte Arten, wie Leucoptera meyricki, Leucoptera coma und Leucoptera caffeina, sind in Zentralafrika beheimatet; vgl. Kranz, Schmutterer, Koch, 1977: Diseases, pests and weeds in tropical crops. Hill 1975: Agricultural Pests of the tropics and their control.

Die wirtschaftliche Bedeutung der Schädlinge besteht darin, daß durch die minierende Lebensweise der Larven in der Palisadenschicht der Blätter erhebliche Anteile der assimilatorischen Oberfläche zerstört werden können. Blattfall, insbesondere wenige Wochen vor der Ernte der Kaffeebeeren, führt zu starken Ertragsausfällen. Eine weitere Folge der Blattschädigung ist die Schwächung der Fruchtäste oder der ganzen Bäume, die sich erst in der nächsten Saison erholen.

Die Witterung hat großen Einfluß auf die Entwicklung des Schädlings. Trockenheit wirkt befallsbegünstigend, mehrere Generationen pro Saison sind möglich.

Die Bekämpfung der Miniermotten ist aufgrund ihrer versteckten Lebensweise - die Jungraupen minieren getrennt in runden Einzelminen, die Verpuppung erfolgt blattunterseits in einem Kokon - nicht einfach. Eingesetzt werden können insektizide Wirkstoffe aus der Gruppe der Phosphorsäureester mit ausgeprägter Tiefenwirkung und Pyrethroide. Sämtliche derartige Produkte haben jedoch den Nachteil, daß sie keine selektive Wirkung zeigen und in vielen Fällen auch warmblütertoxisch sind.

Eine zuverlässige Methode zur Erkennung des Auftretens dieses schädlichen Schmetterlings oder eine selektive Bekämpfungsmethode waren aber bisher nicht bekannt und somit eine gezielte Bekämpfung nicht möglich.

Es ist an sich bekannt, daß bei Schmetterlingen von paarungsbereiten weiblichen Tieren Sexuallockstoffe (= Pheromone) erzeugt und in die Umgebung ausgeschieden werden; männliche Schmetterlinge derselben Art können dann mit Hilfe dieses Riechstoffes die Weibchen finden.

Grundsätzlich gibt es drei verschiedene Möglichkeiten, Sexuallockstoffe im Pflanzenschutz anzuwenden:

Sogenannte Pheromonfallen, bestückt mit synthetischen Sexuallockstoffködern, werden in potentiellen Befallsgebieten ausgehängt. Der Fallenfang von männlichen Faltern erbringt den Nachweis des Auftretens des Schädlings. Außerdem lassen sich Hinweise zur Befallstärke und auf den richtigen Zeitpunkt der Bekämpfung ableiten.

Man kann den Lockstoff mit insektiziden Wirkstoffen kombinieren. Es besteht die Möglichkeit, dem Köder/der Falle, Insektizide zuzusetzen oder aber nur in unmittelbarer Umgebung der Falle zu behandeln. Damit dann der größte Teil der aus weiter Entfernung angelockten männlichen Falterbevölkerung abgetötet werden (Abfangtechnik). Die Biotopbelastung ist auf ein vertretbares Maß reduziert.

Schließlich kann der Schädling durch das Verfahren der Luftraumsättigung mit Sexuallockstoffen oder ähnlich wirkenden Substanzen bekämpft werden. Die männlichen Schmetterlinge werden am Auffinden der Weibchen gestört und somit die Paarung der Tiere verhindert. In diesem Fall wird im gesamten Bereich der zu schützenden Pflanzenkultur eine größere Menge des Lockstoffes gleichmäßig im Luftraum verteilt, so daß die Männchen überall die Gegenwart des Duftstoffes empfinden können und ihr normales Orientierungsverhalten gestört ist.

Selbst bei dieser letztgenannten Verfahrensweise der Anwendung von Sexuallockstoffen werden nur verhältnismäßig kleine Mengen der Wirkstoffe, welche oft nur Bruchteilen der üblichen Dosen der klassischen Insektizidwirkstoffe entsprechen, benötigt (vgl. Birch ed., Pheromones, North Holland Publ. Co., 1974).

Es handelt sich dabei um eine äußerst selektive, untoxische Bekämpfungsmethode unter größtmöglicher Schonung der Nicht-Zielorganismen, insbesondere der Nützlinge.

Es wurde nun gefunden, daß Zubereitungen, die 5,9-Dimethylpentadecan (I) und/oder 5,9-Dimethylhexadecan (II) enthalten, einen wirksamen Lockstoff zur Anwendung, auf Perileucoptera coffeella darstellen.

Gegenstand der Erfindung sind somit Mittel zur Anlockung oder zur Verwirrung männlicher Tiere, der Art Perileucoptera coffeella, die 5,9-Dimethylpentadecan (I) und/oder 5,9-Dimethylhexadecan (II) enthalten.

Ein möglicher Syntheseweg für die neuen Verbindungen ist der im folgenden Herstellungsbeispiel beschriebene. Er führt bei Wahl racemischer Ausgangsstoffe zur entsprechenden Gemischen; aus sterisch einheitlichen Ausgangsstoffen werden entsprechende Stereoisomere erhalten.

Als Baustein wird 1-Brom-3-chlor-2-methylpropan verwendet; es wird in der ersten Stufe mit Propylmagnesiumhalogenid in Gegenwart eines Dialkalimetall-tetrahalogencuprates, vorzugsweise von Dilithiumtetrachlorocuprat, in das 1-Chlor-2-methylhexan überführt. 1-Chlor-2-methylhexan wird durch Behandlung mit Magnesium und anschließendes Umsetzen mit Formaldehyd in das 3-Methylheptanol-(1) überführt. Hieraus wird durch ein gängiges Bromierungsverfahren, z.B. Bromwasserstoffsäure, Phosphortribromid, Triphenylphosphin/Brom/Triphenylphosphin/Tetrabrommethan oder Triphenylphosphin/N-Bromsuccinimid das 1-Brom-3-methylheptan vorgestellt. Es ist gleichermaßen möglich, durch Chlorierung die entsprechende Chlorverbindung zu gewinnen.

Die Halogenverbindung wird mit Magnesium in die entsprechende Grignardverbindung überführt, die, wiederum in Gegenwart z.B. von Dilithiumtetrachlorocuprat, mit 1-Brom-3-chlor-2-methylpropan zum 1-Chlor-2,6-dimethyldecan umgesetzt wird.

Über die Grignardverbindung wird nunmehr entweder mit n-Pentylbromid zum (5 R, S; 9 R, S)-5,9-Dimethylpentadecan oder mit n-Hexylbromid zum (5 R,S; 9 R,S)-5,9-Dimethylhexadecan umgesetzt.

Die Reihenfolge der vorstehend beschriebenen Schritte kann im übrigen mit den fachüblichen Überlegungen auch vertauscht werden.

Herstellungsbeispiel

1-Chlor-2-methylhexan 3

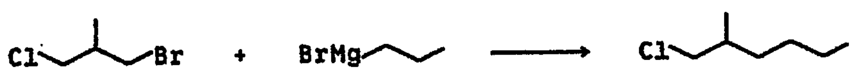

$C_4H_8BrCl$/171,5 (1) (2) $C_7H_{15}Cl$/134,5 (3)

29,3 g (1,21 Mol) Magnesium-Späne werden in 140 ml THF vorgelegt. Bei +40 - 50°C wird 147,6 g (1,2 Mol) 1-Brompropan in 300 ml THF zugetropft, nochmals 200 ml THF zugegeben und 1 h bei +40°C nachgerührt. Dann kühlt man auf -25°C ab, setzt 103 g (0,60 Mol) 1-Brom-3-chlor-2-methylpropan 1 in 300 ml THF und anschließend eine 1-molare Lösung von Dilithiumtetrachlorocuprat in THF (31,6 ml) zu. Man rührt jeweils 2 h bei -20°C, 2 h bei 0°C und über Nacht bei Raumtemperatur. Unter Kühlung im Eiswasserbad wird mit gesättigter Ammoniumchloridlösung hydrolysiert, mehrmals mit Ether extrahiert, mit gesättigter Ammoniumchloridlösung gewaschen, über Natriumsulfat getrocknet und eingeengt.
Auswaage: 75,4 g
Destillation bei 30 mbar/51°C liefert 58 g der Verbindung (3) mit 98 - 99%iger Reiheit.
Ausbeute: 71,9% der berechneten Theorie.

3-Methylheptanol-(1) (4)

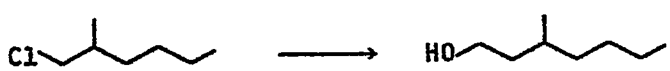

$C_7H_{15}Cl$/134,5 (3) $C_8H_{18}O$/130 (4)

2,0 g (82,3 mg-Atom) Magnesium-Späne werden mit Jod angeätzt. Nach Zugabe von 1 ml einer Lösung von 11 g 96%igem 1-Chlor-3-methylhexan 3 (78,5 mmol) in 10 ml THF bei +60°C wird durch Zugabe von 0,2 ml 1,2-Dibromethan gestartet. Dann wird das restliche 3 zugetropft. Man verdünnt mit 15 ml THF und rührt 2,5 h unter Rückfluß. Bei 40 - 50°C wird anschließend gasförmiger Formaldehyd eingeleitet. Man rührt 1 h bei Raumtemperatur nach, hydrolysiert durch Zugabe von 10 ml 30%iger Schwefelsäure und destilliert mit Wasserdampf. Das Destillat wird mehrmals mit Ether extrahiert, sodann mit Kochsalzlösung gewaschen, über Natriumsulfat getrocknet und eingeengt.
Rückstand: 10,8 g mit einem Gehalt an (4) von 74%.
Ausbeute: 78,3%.

Man destilliert bei 0,3 mbar bis zu einer Übergangstemperatur von 80°C und erhält 7,95 g der Verbindung (4); Reinheit 95%; Ausbeute 74,0%.

1-Brom-3-methylheptan (5)

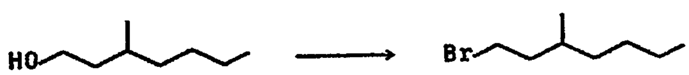

$C_8H_{18}O$/130 (4) $C_8H_{17}Br$/193 (5)

7,7 g 95%iges 3-Methylheptanol-(1) 4 (56,3 mmol) werden vorgelegt, bei 0°C 10,44 g (38,5 mmol) Phosphortribromid zugetropft und 5 h bei 100° gerührt. Man läßt abkühlen, gießt auf Eiswasser, extrahiert mehrmals mit Hexan, wäscht mit Sodalösung, trocknet über Magnesiumsulfat und engt ein.
Auswaage: 11,2 g mit einem gaschromatographisch bestimmten Gehalt an (5) von 93% (Ausbeute 95,9%).

Nach Destillation bei 30 mbar/110°C (Kugelrohr) erhält man 10,31 g 5 mit einer Reinheit von 97% (Ausbeute 92,0%).

1-Chlor-2,6-dimethyldecan (6)

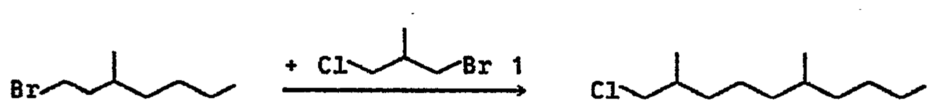

$C_8H_{17}Br/193$ (5)    $C_{12}H_{23}Cl/202,5$ (6)

1,14 (47 mmol) Magnesium werden in 7 ml THF vorgelegt, bei 50°C 9 g 1-Brom-3-methylheptan 5 (97%ig; ≙ 45,2 mmol) in 16 ml THF zugetropft und 1 h bei +40°C nachgerührt. Man kühlt auf -30°C ab, gibt zuerst 6,8 g 1-Brom-3-chlor-2-methylpropan 1 (39,7 mmol) in 8 ml THF und anschließend 5,5 ml einer 0,5molaren Lösung von Dilithiumtetrachlorocuprat in THF zu. Man rührt 2 h bei -30°C, 2 h bei 0°C und dann über Nacht bei Raumtemperatur. Unter Kühlung im Eiswasserbad wird durch Zugabe von gesättigter Ammoniumchloridlösung hydrolysiert, mehrmals mit Ether extrahiert, mit gesättigter Ammoniumchloridlösung gewaschen, über Magnesiumsulfat getrocknet und eingeengt. Der Rückstand wird bei 0,5 mbar bis 120°C destilliert. Man gewinnt 5,38 g 6 mit einer Reinheit von 93% (Ausbeute von 62,2%, bezogen auf 1).

5,9-Dimethylpentadecan

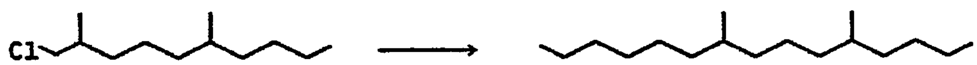

$C_{12}H_{23}Cl/202,5$ (6)    $C_{17}H_{36}/240$ (5,9-Dimethyl-$C_{15}$)

0,54 g (22,2 mmol) Magnesium werden mit Jod angeätzt, nach Zugabe von 0,5 ml einer Lösung von 4,5 g 93%igem (20,7 mmol) 1-Chlor-2,6-dimethyldecan (6) in 4 ml THF bei +60°C durch Zugabe eines Tropfens 1,2-Dibromethan gestartet und das restliche (6) zugetropft. Man verdünnt mit 4 ml THF und rührt 2,5 h unter Rückfluß. Dan wird auf -20°C abgekühlt, zunächst 3,8 g (25 mmol) n-Pentylbromid 7 in 4 ml THF und anschließend 3 ml einer 0,5molaren Lösung von Dilithiumtetrachlorocuprat in THF zugesetzt. Man rührt 2 h bei -20°C, 2 h bei 0°C und dann über Nacht bei Raumtemperatur. Nach Aufarbeitung mit Ammoniumchloridlösung und Destillation (Kugelrohr; 130°C/0.3 mbar), erhält man 3,22 g 5,9-Dimethylpentadecan entsprechend einer Ausbeute von 64,8%, bezogen auf eingesetztes 6.

Zur erfindungsgemäßen Formulierung des Wirkstoffs kommen sowohl flüssige wie auch feste Präparationen in Frage.

Als Lösungsmittel kommen hochsiedende, aromatische, aliphatische oder cycloaliphatische Verbindungen in Betracht. Neben Kohlenwasserstoffen eignen sich Ester, Ether oder Ketone besonders gut. Typische Vertreter dieser Klassen sind z.B. Xylol, Methylnaphthaline, Paraffinöle, Cyclohexanon, Ethylglykolacetat, Isophoron und Dibutylphthalat.

Zum Zwecke der Wirkungsverlängerung können Lösungen in pflanzlichen, tierischen oder synthetischen Ölen oder Fetten und anderen verdunstungshemmenden Lösungsmitteln mit niedrigem Dampfdruck (wie z.B. Dioctylphthalat) hergestellt werden.

Feste Zubereitungen erhält man dadurch, daß man den Wirkstoff in oder an natürliche oder synthetische feste Träger wie Gummi, Kork, Zellulose, Kunststoffe, gemahlene Kohle, Holzmehl, Silikate, Bimskies, gebrannten Ton oder ähnliche feste Trägerstoffe bindet; in Kunststoffkapseln oder -behältern sowie in mehrschichtigen Kunststoffplättchen, sogenannten Flakes, eingeschlossener Wirkstoff kann durch die Wände diffundieren; auch dadurch wird eine gleichmäßige Angabe an die Luft über längere Zeiträume hinweg erreicht. Außerdem kann der Wirkstoff aus geeigneten Behältern (Kapillaren oder anderen Gefäßen) durch enge Öffnungen zur Verdunstung gebracht werden, wodurch über längere Zeiträume hinweg besonders gleichmäßig Duftkonzentrationen erzielt werden.

Der Gehalt dieser Zubereitungen an Wirkstoff kann innerhalb weiter Grenzen schwanken. Generell kann das Verhältnis Wirkstoff : Zusatzstoff z.B. im Bereich von z.B. 10:1 bis 1:$10^3$ liegen. In Kapselformulierungen oder anderen geeigneten Behältern kann z.B. Wirkstoff in reiner, unverdünnter Form angewendet werden und sein Gewichtsanteil, bezogen auf die Gesamtformulierung, sehr hoch sein und bis zu 90% betragen. Im allgemeinen genügen jedoch sehr geringe Wirkstoffkonzentrationen in den Zubereitungen, um die gewünschte Wirkung auf Leucoptera-Männchen auszuüben. Bevorzugt ist ein Mengenverhältnis Wirkstoff : Zusatzstoff von 1:3 bis 1:$10^2$.

Man kann den Wirkstoff auch in vergleichsweise hohen Konzentrationen ausbringen, und die Männchen durch Desorientierung und Konfusion nicht nur am Auffinden der Weibchen, sondern auch unmittelbar an der Paarung hindern. Für diese Methode eignen sich am besten Formulierungen mit schwerflüchtigen Zusatzstoffen, die den Wirkstoff protrahiert abgeben, wie Gummi, Zellstoff, Wachse, Polymerisate oder verdunstungshemmende, schwerflüchtige Öle der Paraffine, sowie Formulierungen in Kapseln oder anderen Behältern (Kapillaren), die den Lockstoff entweder durch ihre Wandung oder durch enge Öffnungen abgeben. Die Wirkstoffkonzentration liegt hier im allgemeinen im Bereich von 10:1 bis 1:$10^3$.

Freilanduntersuchungen in einem potentiellen Befallsgebiet von Perileucoptera coffeella belegen die biologische Funktion der beanspruchten Pheromonkomponente.

## Patentansprüche

1. 5,9-Dimethylpentadecan (I).
2. 5,9-Dimethylhexadecan (II).
3. Mittel zur Bekämpfung der Kaffeeminiermotte Perileucoptera coffeella, enthaltend 5,9-Dimethylpentadecan und/oder 5,9-Dimethylhexadecan als Lockstoff.
4. Mittel nach Anspruch 3, enthaltend übliche Zusatzstoffe und Hilfsmittel.
5. Verfahren zur Bekämpfung der Kaffeeminiermotte, dadurch gekennzeichnet, daß man ein Mittel nach Anspruch 3 in Verbindung mit einem Insektizid anwendet.
6. Verfahren zur Bekämpfung der Kaffeeminiermotte, dadurch gekennzeichnet, daß man mit einem Mittel nach Anspruch 3 das Auftreten der Kaffeeminiermotte in an sich bekannter Weise feststellt und danach an sich bekannte Bekämpfungsmethoden anwendet.
7. Verfahren zur Beeinflussung der Vermehrungsrate der Kaffeeminiermotte, dadurch gekennzeichnet, daß man ein Mittel nach Anspruch 3 in einer solchen Menge anwendet, daß die männlichen Tiere der Art bei der Auffindung der weiblichen Tiere gestört werden.

## Claims

1. 5,9-Dimethylpentadecane (I).
2. 5,9-Dimethylhexadecane (II).
3. An agent for controlling the coffee leaf miner Perileucoptera coffeella, containing 5,9-dimethylpentadecane and/or 5,9-dimethylhexadecane as an attractant.
4. An agent as claimed in claim 3, containing conventional additives and assistants.
5. A method for controlling the coffee leaf miner, wherein an agent as claimed in claim 3 is used in conjunction with an insecticide.
6. A method for controlling the coffee leaf miner, wherein the occurrence of the coffee leaf miner is established in a conventional manner using an agent as claimed in claim 3, after which conventional control methods are used.
7. A method for influencing the multiplication rate of the coffee leaf miner, wherein an agent as claimed in claim 3 is used in an amount such that the males of the species are disturbed during their search for the females.

## Revendications

1. Le 5,9-diméthylpentadécane (I).
2. Le 5,9-diméthylhexadécane (II).
3. Produit pour combattre la mineuse du café Perileucoptera coffeella; contenant du 5,9-diméthylpentadécane et/ou du 5,9-diméthylhexadécane en tant qu'appât.
4. Produit selon la revendication 3, contenant des additifs et produits auxiliaires usuels.
5. Procédé pour combattre la mineuse du café Perileucoptera coffeella, caractérisé en ce que l'on utilise un produit selon la revendication 3 en combinaison avec un insecticide.
6. Procédé pour combattre la mineuse du café Perileucoptera coffeella, caractérisé en ce que l'on détecte de manière connue en soi la présence de Perileucoptera coffeella à l'aide d'un produit selon la revendication 3 puis on applique des procédés de lutte connus en soi.
7. Procédé pour agir sur la vitesse de multiplication de la mineuse du café, caractérisé en ce que l'on utilise un produit selon la revendication 3 en quantité telle que la recherche des femelles par les mâles de l'espèce est perturbée.